# EUROPEAN PATENT SPECIFICATION

(11) **EP 0 808 624 B1**
(45) Date of publication and mention of the grant of the patent: **06.12.2000**
(21) Application number: 97106650.1
(22) Date of filing: 22.04.1997
(51) Int. Cl.: A61K 7/06

(54) **Hair tonic**
Haartonikum
Lotion capillaire

(30) Priority: 22.05.1996 DE 19620662; 25.03.1997 DE 19712555
(43) Date of publication of application: 26.11.1997
(73) Proprietor: Kao Corporation, Tokyo 103-8210 (JP)
(72) Inventor: Dubowoj, Polina, 64319 Pfungstadt (DE)
(74) Representative: Kindler, Matthias, Dr. Dipl.-Chem.

(56) References cited:
- US-A- 4 798 721
- PATENT ABSTRACTS OF JAPAN vol. 011, no. 060 (C-405), 24 February 1987 (1987-02-24) & JP 61 218508 A (SHOWA DENKO KK), 29 September 1986 (1986-09-29)

## Description

This invention comprises a composition for the treatment of hair and scalp in the form of a hair tonic showing an attractive product appearance with good applicability onto hair and scalp performing a pleasant and reviving effect thereon, and, in addition providing volume, combability, a solid touch and lustre to the hair.

Usual hair tonics or lotions are provided as transparent hydro-alcoholic solutions to be massaged into the scalp.

So far, opaque hair lotions with an attractive pearlescent lustre, providing additionally hair conditioning properties, are not known.

Thus, the invention provides a new product having the properties mentioned above.

Accordingly, the object of the invention is an aqueous hair tonic containing at least one lower alcohol, selected from the group of ethyl, n-propyl and (or) isopropyl alcohol, and a combination of
a) 0.05% to 2.5% by wt. of at least one water-soluble anionic methacrylic or acrylic acid homo- or copolymer,
b) 0.05% to 2.5% by wt. of at least one water-insoluble, but water-dispersible polymer,
all percentages calculated to the total composition of this hair tonic.

DE-A 44 14124 describes already hair treatment compositions, especially hair sprays, which are aqueous dispersions of insoluble polymers with a glass transition temperature between -20 and +70°C, also containing a water-soluble polymer which is supposed to increase the rinsability of said product out of the hair.
Those water-soluble polymers are preferably nonionic or zwitterionic; the "Ultrahold® strong" acrylic acid/ethyl acrylate/N-tert. butyl acrylamide terpolymer mentioned there is not water-soluble and thus is not suitable for use in the hair tonic according to the invention.

The proportion of the lower alcohol in the hair tonic according to the invention is preferably from about 5% to 75%, particularly 10% to 60%, preferably about 15% to 50% by wt., calculated to the total composition.

The water-soluble anionic methacrylic and acrylic acid homo- and copolymers are preferably applied in a proportion of 0.1% to 1%, especially 0.15% to 0.75% by wt., calculated to the total composition of the hair tonic

These polymers may be completely or partially neutralized and be present as sodium or ammonium salts or amine salts, e.g., mono-, di- or triethanolamine salts. They may also be crosslinked provided that they maintain their water-solubility.

Suitable acrylic acid polymers are well-known on the market under the trade names "Carbopol®", e.g., C. 934, C. 940, C. 941 and C. 1342, "Acrysol®", "Acritamer®", but also under the CTFA name "Carbomer".

As water-insoluble, though water-dispersible polymers styrene homo- and copolymers are especially useful within the scope of the invention, e.g., styrene/vinyl pyrrolidone copolymers of the type "Antara® 430" or "Polectron® 430", styrene/acrylic acid ester copolymers, e.g. a styrene/butyl acrylate copolymer of the type "Acronal®", styrene/alkyl acrylate/(meth)acrylic acid copolymers, e.g., "Syntran® 5002", styrene/acrylamide copolymers or water-dispersible styrene/alkyl(meth)acrylate/acrylonitrile/(meth)acrylic acid copolymers.

Applicable are also water-insoluble, but water-dispersible alkyl(meth)acrylate/acrylamide copolymers, alkyl(meth)acrylate/vinyl pyrrolidone copolymers or partially saponified vinyl acetate/alkyl acrylate copolymers.

The preferred quantity of the water-insoluble, water-dispersible polymers in the hair tonic according to the invention is between about 0.1% to 1%, particularly from 0.15% to 0.75% by wt., of the total composition.

If the water-insoluble polymer is added to the hair lotion as an aqueous dispersion, the quantities of course refer to the content of polymers in this dispersion, calculated to the final composition.

In addition to the essential ingredients mentioned above, the hair lotions according to the invention may also contain any desired type of actives and additives.

These are particularly plant oils in low quantities, e.g., 0.01% to about 2.5% by wt., calculated to the total composition, e.g. 0.01% to about 1% by wt. wheat germ oil.

In this regard, the additional use of an appropriate amount of emulsifiers is recommendable.

Further suitable additives are moisturizers such as polyalcohols, e.g.,1,2-propanediol and glycerol, pyrrolidone carboxylic acid, fruit acids such as lactic acid, citric acid, tartaric acid, glycolic acid, pyruvic acid and malic acid, panthenol and lactylates, e.g. isostearoyl lactylate.

Particularly suitable actives are plant extracts, e.g., oatmeal extract (from Avena sativa) having an antipruritic effect, camomile extract, lime blossom extract, horse chestnut extract, whitethorn extract, etc., also other ingredients stimulating blood circulation and (or) having an antiphlogistic effect.

Perfumes and dyestuffs may be added, also preservatives unless the content of antimicrobial ingredients such as ethanol does not provide enough protection anyhow.

Suitable formulae of compositions for hair and scalp lotions and a list of customary ingredients contained therein is given in the monography of K. Schrader, "Grundlagen und Rezepturen der Kosmetika", 2nd Ed. (1989, Hüthig Buch Verlag, Heidelberg), pp. 763 to 771, which is incorporated by reference.

The production of the hair lotions according to the invention is performed by a usual process of mixing all ingredients, optionally including pre-mixtures or pre-emulsions.

The following examples illustrate the invention.

### Example 1

| Hair tonic for dry scalp | |
|---|---|
| Allantoin | 0.10 (% by wt.) |
| Oatmeal extract (from Avena sativa) | 0.50 |
| Tocopherol acetate | 0.10 |
| Wheat germ oil | 0.01 |
| Tartaric acid | 0.01 |
| Citric acid | 0.01 |
| Polypropylene glycol (PPG-420) | 0.10 |
| Isostearoyl lactylate | 0.05 |
| Acrylic acid/C₁₀-C₃₀-alkyl acrylate copolymer (Carbopol® 2020) | 0.15 |
| Styrene/vinyl pyrrolidone copolymer (Antara® 430) | 0.30 |
| Benzophenone | 0.10 |
| Ethanol | 25.00 |
| Sodium hydroxide solution 32% | 0.07 |
| PEG-60 Hydrogenated castor oil | 0.25 |
| Perfume | 0.10 |
| Dyestuffs | q.s. |
| Water | @ 100.00 |

In a double blind test (half-side testing) on 10 volunteers, one side of hair and scalp was treated with a composition according to Example 1 and the other side with an identical composition 1A which did not contain styrene/vinyl pyrrolidone copolymer.

Thereafter an assessment was made by two hairdressers who, independently from each other, evaluated the hair properties of the two compositions. The results are shown in the following table:

| | **Composition 1** | **Composition 1A** |
|---|---|---|
| Appearance | opaque, lustrous | inattractive |
| Applicability | good | sticky, soft |

| | **Preferential values:** | |
|---|---|---|
| Volume of the hair | 18 | 2 |
| Combability | 20 | 0 |
| Volume and body of the hair | 20 | 0 |
| Lustre | 18 | 2 |

These results show the unexpected superiority of the hair tonic according to the invention.

### Example 2

| Hair tonic for sensitive scalp | |
|---|---|
| Panthenol | 0.10 (% by wt.) |
| Isostearoyl lactylate | 0.10 |
| Oatmeal extract (from Avena sativa) | 0.50 |
| Lime blossom extract (hydro-alcoholic) | 0.10 |
| 1,2-Propylene glycol | 0.50 |
| Allantoin | 0.05 |
| Citric acid | 0.05 |
| Glycolic acid | 0.01 |
| Malic acid | 0.02 |
| Tartaric acid | 0.02 |
| Pyrrolidone carboxylic acid | 0.03 |
| Styrene/vinyl pyrrolidone copolymer (Antara® 430) | 0.45 |
| Crosslinked, partially neutralized acrylic acid copolymer (Carbopol® 934) | 0.20 |
| Sodium hydroxide solution, 32% | 0.08 |
| PEG-60 Hydrogenated castor oil | 0.25 |
| Perfume | 0.05 |
| Dyestuffs | q.s. |
| Water | @ 100.00 |

## Claims

1. Aqueous lotion for the treatment of human hair and scalp, comprising at least one lower alcohol, selected from the group of ethyl, n-propyl and (or) isopropyl alcohol, and a combination of
a) 0.05% to 2.5% by wt. of at least one water-soluble anionic methacrylic acid or acrylic acid homo- or copolymer and
b) 0.05% to 2.5% by wt. of at least one water-insoluble, but water-dispersible polymer, all percentages calculated to the total composition.

2. Lotion according to claim 1, comprising 0.1% to 1% by wt., calculated to the total composition, of at least one water-soluble anionic methacrylic acid or acrylic acid homo- or copolymer.

3. Lotion according to claims 1 or 2, comprising as ingredient b) 0.1% to 1% by wt. of at least one water-insoluble polymer.

4. Lotion according to one or more of claims 1 to 3, comprising as ingredient b) at least one water-insoluble styrene homo- or copolymer.

5. Lotion according to claim 4, comprising as ingredient b) a water-insoluble styrene-vinyl pyrrolidone copolymer.

6. Lotion according to one or more of claims 1 to 5, comprising 0.01% to 2.5% by wt., calculated to the total composition, of at least one plant oil.

7. Lotion according to claim 6, comprising 0.01% to 1% by wt. wheat germ oil, calculated to the total composition.

8. Lotion according to one or more of claims 1 to 7, characterized by a pH-value from 4 to 7.

9. Use of an aqueous composition as hair tonic for the treatment of human hair and scalp, comprising at least one lower alcohol, selected from the group of ethyl, n-propyl and (or) isopropyl alcohol, and a combination of
a) 0.05% to 2.5% by wt. of at least one water-soluble, anionic methacrylic or acrylic acid homo- or copolymer,
b) 0.05% to 2.5% by wt. of at least one water-insoluble, but water dispersible polymer, all percentages calculated to the total composition.

## Patentansprüche

1. Haarwasser zur Behandlung von menschlichem Haar und Kopfhaut auf Basis einer wässrigen Grundlage, enthaltend mindestens einen niedrigen Alkohol, ausgewählt aus der Gruppe Ethyl-, n-Propyl- und /oder Isopropylalkohol, und eine Kombination aus
a) 0,05 bis 2,5 Gew.% mindestens eines wasserlöslichen anionischen Methacrylsäure- oder Acrylsäurehomo- oder -copolymers, und
b) 0,05 bis 2,5 Gew.% mindestens eines wasserunlöslichen, jedoch wasserdispergierbaren Polymers, jeweils berechnet auf die Gesamtzusammensetzung.

2. Haarwasser nach Anspruch 1, enthaltend 0,1 bis 1 Gew.%, berechnet auf die Gesamtzusammensetzung, mindestens eines wasserlöslichen, anionischen Methacrylsäure- oder Acrylsäurehomo- oder -copolymers.

3. Haarwasser nach Anspruch 1 oder 2 , enthaltend als Bestandteil b) 0,1 bis 1 Gew.% mindestens eines wasserunlöslichen Polymers.

4. Haarwasser nach einem oder mehreren der Ansprüche 1 bis 3, enthaltend als Bestandteil b) mindestens ein wasserunlösliches Styrolhomo- oder -copolymer.

5. Haarwasser nach Anspruch 4, enthaltend als Bestandteil b) ein wasserunlösliches Styrol-Vinylpyrrolidon-Copolymer.

6. Haarwasser nach einem oder mehreren der Ansprüche 1 bis 5, enthaltend 0,01 bis 2,5 Gew.%, berechnet auf die Gesamtzusammensetzung, mindestens eines pflanzlichen Öls.

7. Haarwasser nach Anspruch 6, enthaltend 0,01 bis 1 Gew.%, berechnet auf die Gesamtzusammensetzung, Weizenkeimöl.

8. Haarwasser nach einem oder mehreren der Ansprüche 1 bis 7, gekennzeichnet durch einen pH-Wert von 4 bis 7.

9. Verwendung einer wässrigen Zusammensetzung, enthaltend mindestens niederen Alkohol, ausgewählt aus der Gruppe Ethyl-, n-Propyl- und/oder Isopropylalkohol, und eine Kombination aus
a) 0,05 bis 2,5 Gew.% mindestens eines wasserlöslichen, anionischen Methacrylsäure- oder Acrylsäurehomo- oder -copolymers, und
b) 0,05 bis 2,5 Gew.% mindestens eines wasserunlöslichen, jedoch wasserdispergierbaren Polymers, jeweils berechnet auf die Gesamtzusammensetzung, als Haarwasser zur Behandlung des menschlichen Haares und der Kopfhaut.

## Revendications

1. Lotion aqueuse pour le traitement des cheveux et du cuir chevelu humains, comprenant au moins un alcool inférieur choisi dans le groupe constitué de l'alcool éthylique, l'alcool n-propylique et/ou l'alcool isopropylique, et une combinaison de
a) 0,05 % à 2,5 % en poids d'au moins un acide méthacrylique anionique ou d'un homo- ou copolymère d'acide acrylique anionique soluble dans l'eau, et
b) 0,05 % à 2,5 % en poids d'au moins un polymère insoluble dans l'eau mais dispersible dans l'eau, tous les pourcentages étant rapportés à la composition totale.

2. Lotion selon la revendication 1, comprenant 0,1 % à 1 % en poids, rapporté à la composition totale, d'au moins un acide méthacrylique anionique ou d'un homo- ou copolymère d'acide acrylique anionique soluble dans l'eau.

3. Lotion selon la revendication 1 ou 2, comprenant en tant qu'ingrédient b) 0,1 % à 1 % en poids d'au moins un polymère insoluble dans l'eau.

4. Lotion selon une ou plusieurs des revendications 1 à 3, comprenant en tant qu'ingrédient b) au moins un homo- ou copolymère de styrène insoluble dans l'eau.

5. Lotion selon la revendication 4, comprenant en tant qu'ingrédient b) un copolymère de styrène-vinylpyrrolidone insoluble dans l'eau.

6. Lotion selon une ou plusieurs des revendications 1 à 5, comprenant 0,01 % à 2,5 % en poids, rapporté à la composition totale, d'au moins une huile végétale.

7. Lotion selon la revendication 6, comprenant 0,01 % à 1 % en poids, rapporté à la composition totale, d'huile de germe de blé.

8. Lotion selon une ou plusieurs des revendications 1 à 7, caractérisée par un pH compris entre 4 et 7.

9. Utilisation d'une composition aqueuse en tant que tonique capillaire pour le traitement des cheveux et du cuir chevelu humains, comprenant au moins un alcool inférieur choisi dans le groupe constitué de l'alcool éthylique, l'alcool n-propylique et/ou l'alcool isopropylique, et une combinaison de
a) 0,05 % à 2,5 % en poids d'au moins un homo- ou copolymère d'acide acrylique ou méthacrylique anionique soluble dans l'eau, et
b) 0,05 % à 2,5 % en poids d'au moins un polymère insoluble dans l'eau mais dispersible dans l'eau, tous les pourcentages étant rapportés à la composition totale.
